# EUROPÄISCHE PATENTSCHRIFT

(11) **EP 2 129 465 B1**
(45) Veröffentlichungstag und Bekanntmachung des Hinweises auf die Patenterteilung: **03.08.2011**
(21) Anmeldenummer: 08734751.4
(22) Anmeldetag: 25.03.2008
(51) Int. Cl.: B01L 3/02, B01L 3/14

(54) **VERFAHREN UND VORRICHTUNG ZUM DOSIERTEN AUSBRINGEN EINES MEDIUMS**
METHOD AND DEVICE FOR THE METERED DISPENSING OF A MEDIUM
PROCÉDÉ ET DISPOSITIF POUR LA DISTRIBUTION DOSÉE D'UN MILIEU

(30) Priorität: 22.03.2007 DE 102007014418
(43) Veröffentlichungstag der Anmeldung: 09.12.2009
(73) Patentinhaber: SCHLIEMANN, Eric, 78256 Steisslingen (DE)
(72) Erfinder: SCHLIEMANN, Eric, 78256 Steisslingen (DE)
(74) Vertreter: Weiss, Peter
(86) Internationale Anmeldenummer: PCT/EP2008/002344
(87) Internationale Veröffentlichungsnummer: WO 2008/113611

(56) Entgegenhaltungen:
- EP-A- 0 117 489
- EP-A- 0 250 095
- WO-A-00/18453
- WO-A-2005/087127
- DE-A1- 2 533 036
- FR-A- 2 493 515
- US-A- 3 101 751
- US-A- 3 672 369
- US-A- 4 614 267

## Beschreibung

Die Erfindung betrifft eine Dosierpipette zum dosierten Ausbringen eines Mediums aus einem Behälter mit einem Dosiergehäuse in dem ein Kolben begrenzt axial verschiebbar sitzt, wobei diese Hubbegrenzung durch Drehen des Kolbens veränderbar ist.

### STAND DER TECHNIK

In vielen Fällen des täglichen Lebens oder auch bei industriellen Anwendungen muss ein Medium, insbesondere Flüssigkeit, aus einem Behälter entnommen werden. Dies gilt vor allem auch im medizinischen Bereich. Hier ist allgemein eine Pipette bekannt, welche in den Behälter eingesetzt wird, wobei durch Drücken und Loslassen eines Gummielementes das Medium in ein Röhrchen eingesaugt wird. Dabei ist es nicht möglich, das zu entnehmende Medium zu dosieren.

Aus der WO2004/054720 ist beispielsweise eine Dosiervorrichtung zum Ansaugen und Ausbringen eines fliessfähigen Mediums aus einem Behälter mit einem Einlass und einem Durchlass bekannt, wobei Einlass und Durchlass voneinander getrennt angeordnet sind und zwischen ihnen eine Dosier- und Verdrängungskammer vorgesehen ist. Sowohl Einlass wie auch Durchlass sind verschliessbar. Dabei ist diese Dosiervorrichtung aber bleibender Bestandteil des Behälters selbst und kann von diesem nicht getrennt gehandhabt werden.

Auch aus der WO 00118453 ist eine Dosiervorrichtung bekannt. Diese umfasst aber ebenfalls neben der Entnahmevorrichtung einen Behälter, die nur in Kombination miteinander verwendet werden können. Zur Einstellung der gewünschten Menge, kann an einem Rad, das sich über dem Behälterverschluss befindet, gedreht werden.

Aus der FR 2 493 515 A geht ebenfalls eine Dosiervorrichtung hervor, mit welcher ein gasförmiges, pastöses oder flüssiges Produkt aus einem Behälter entnommen werden kann. Mittels einer begrenzbaren Kolbenbewegung kann die Dosis des Produktes gesteuert werden. Hierzu ist am Kolben ein Ausschnitt vorgesehen, welcher unterschiedlich axial abgestufte Begrenzungen aufweist, die mit einem Führungssteg in Eingriff gelangen. Ein weiterer Führungssteg ist dabei auf einer gegenüberliegenden Seite des Kolbens vorgesehen, um ein Abgleiten des Nockens aus den axialen Begrenzungen zu verhindern.

### AUFGABE

Aufgabe der vorliegenden Erfindung ist es, ein dosiertes Entnehmen eines Mediums aus einem Behälter und die getrennte Handhabung dieser Entnahmevorrichtung von dem Behälter zu ermöglichen. Ferner soll der Langzeiteinsatz der Vorrichtung verbessert und Fehldosierungen vermieden werden und ein nur wenige Bauteile umfassender Aufbau vorgesehen sein.

### LÖSUNG DER AUFGABE

Zur Lösung dieser Aufgabe führt, dass die Hubbegrenzung durch ein Zusammenwirken eines dem Dosiergehäuse zugeordneten Nockens mit unterschiedlich langen Rinnen erfolgt, welche axial in einen Kolbenmantel des Kolbens eingeformt sind, wobei der Nocken und die Rinnen eine Verdrehsicherung ausbilden.

Das bedeutet, dass die Entnahme des Mediums mit Hilfe einer in den Behälter einsetzbaren Pipette mengenmässig exakt dem Wunsche des Benutzers entspricht. Dieser kann die Entnahmemenge auch, wenn gewünscht, verändern.

Sollte es sich bei dem Behälter beispielsweise um einen verschlossenen Beutel handeln, so wird die Dosierpipette direkt in diesen Beutel eingestochen. In der Regel dürfte es sich bei dem Behälter jedoch um eine Flasche od. dgl. handeln, die mit einem Behälterverschluss versehen ist. In diesem Fall dürfte es ratsam sein, in dem Behälterverschluss eine Öffnung vorzusehen, in die die Pipette eingesetzt werden kann.

Vor dem ersten Einsetzen der Dosierpipette dürfte diese Öffnung von einem Verschlussstopfen belegt sein, damit das im Behälter vorhandene Medium nicht kontaminiert wird. Durch das Einsetzen der Dosierpipette gelangt dieser Verschlussstopfen aus seiner Verschlusslage, wobei er in einem Ausführungsbeispiel der Erfindung über einen Sicherungsbügel mit dem Behälterverschluss verbunden bleibt. In diesem Fall kann der Verschlussstopfen, sofern der Behälterverschluss für eine andere Flasche benutzt werden soll, wieder in Schliesslage verbracht werden.

In einem anderen Ausführungsbeispiel der Erfindung ist der Verschlussstopfen verlierbar in die Öffnung eingesetzt. Er wird über die Dosierpipette in das Innere des Behälters ausgestossen. Sofern er ein ausreichendes Gewicht aufweist, kann dieser Verschlussstopfen jetzt auch als Mischelement für das Medium verwendet werden, wenn beispielsweise der Behälter vor dem Gebrauch geschüttelt werden muss.

Eine erfindungsgemässe Dosierpipette besteht nur aus drei, vorzugsweise aus Kunststoff hergestellten Elementen. Zum einen ist dies das Dosiergehäuse, in das zum anderen der Kolben eingesetzt wird, der wiederum durch das dritte Element, nämlich den Nocken in seiner Hubbewegung gegenüber dem Dosiergehäuse begrenzt wird.

Das Dosiergehäuse ist bevorzugt ein längliches, zylindrisches Röhrchen, welches nach unten ggf. konisch zuläuft und dort eine Ansaugöffnung umschliesst. Nach oben hin ist dieses Röhrchen etwas erweitert und nimmt in einem Ausführungsbeispiel einen Anschlagring auf, von dem nach innen ein Nocken in die lichte Weite einer axialen Ausnehmung des Röhrchens, in der auch der Kolben geführt ist, einragt. Dieser Anschlagring kann in eine entsprechende Ringausnehmung in der Erweiterung eingesetzt sein und wird nach oben hin von einem Bördelrand des Dosiergehäuses übergriffen. Denkbar sind hier aber auch andere Möglichkeiten der Festlegung des Anschlagrings in dem Gehäuse.

In einem anderen Ausführungsbeispiel ist der Nocken ein separates Element, welches in eine radiale Führung in das Dosiergehäuse eingesetzt wird. In dieser radialen Ausnehmung können noch weitere Führungsschienen zum erleichterten Einsetzen des Nockens vorgesehen sein. Ferner befindet sich in der radialen Ausnehmung eine Rasterung, welche von dem Nocken beim Einschieben überfahren wird und zur Verhinderung des Herausgleiten des Nockens aus der Ausnehmung hinter den Nocken einschnappen.

Der Kolben wiederum weist einen Halsbereich auf, der einen geringeren Durchmesser als ein Kolbenmantel hat. Dieser Durchmesser ist so gewählt, dass der Hals beim Drehen des Kolbens an dem Nocken vorbeigleiten kann. Dieser Durchmesser setzt sich dann in Rinnen fort, die in den Kolbenmantel eingeformt sind. D.h., der Nocken kann in diesen Rinnen gleiten, bildet aber dabei gleichzeitig die Verdrehsicherung, so dass, wenn der Nocken in die Rinnen eingefahren ist, nur noch eine axiale Bewegung des Kolbens gegenüber dem Dosiergehäuse möglich ist. Damit der Nocken die ihm zugeordnete Rinne leichter findet, weist diese einen konischen Rinneneinlauf auf.

In den Kolbenmantel sind um die Achse verteilt eine Mehrzahl von Rinnen eingeformt. Diese haben eine unterschiedliche Länge, so dass sie auch einen unterschiedlichen Hub des Kolbens gegenüber dem Dosiergehäuse erlauben. Dieser Hub bestimmt dann die Dosiermenge, welche in einen Dosierraum eingesaugt wird. Dieser Dosierraum wird von einem Endzapfen des Kolbens, einer diesen Endzapfen umfangenden Ringlippe des Kolbens und dem Dosiergehäuse gebildet und erweitert sich beim axialen Bewegen des Kolbens gegenüber dem Dosiergehäuse.

Das Einstellen der Dosiermenge geschieht durch Verdrehen des Kolbens um die Achse, sobald der Nocken im Bereich des Halses des Kolbens liegt. Zum Erkennen der Dosierung sind dabei auf einem Flansch des Dosiergehäuses Markierungen vorhanden, die mit einer Anzeige am Kolben bestimmt werden können. Für den Benutzer bedeutet dies eine sehr leichte Handhabung, die eine Fehldosierung verhindert.

Die gesamte Konstruktion der Dosierpipette ist sehr robust, einfach und kostengünstig herzustellen. Die gesamte Pipette und die dabei verwendeten Materialien sind nach den für die pharmazeutische Industrie geltenden Richtlinien zugelassen, verbraucherfreundlich und erlauben eine saubere Handhabung.

### FIGURENBESCHREIBUNG

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Figur 1: einen Längsschnitt durch eine erfindungsgemässe Dosierpipette;
- Figur 2: eine Draufsicht auf die Dosierpipette gemäss Figur 1;
- Figur 3: einen Aufriss eines Kolbens der Dosierpipette gemäss Figur 1;
- Figur 4 IV-IV;: einen Querschnitt durch den Kolben gemäss Figur 3 entlang Linie
- Figur 5: einen vergrössert dargestellten Querschnitt durch die Dosierpipette gemäss Figur 1 entlang Linie V-V;
- Figur 6: einen Längsschnitt durch ein weiteres Ausführungsbeispiel einer Dosierpipette;
- Figur 7: einen vergrössert dargestellten Teilbereich der Dosierpipette gemäss Figur 6 in Explosionsdarstellung;
- Figur 8: einen schematisch dargestellten Querschnitt durch die Dosierpipette im Teilbereich gemäss Figur 7;
- Figur 9: einen Längsschnitt durch einen nicht zum beanspruchten Gegenstand der vorliegenden Erfindung gehörenden Behälterverschluss;
- Figur 10: einen Längsschnitt durch den Behälterverschluss gemäss Figur 9 mit eingesetzter Dosierpipette in Gebrauchslage;
- Figur 11: einen Längsschnitt durch ein weiteres nicht zum beanspruchten Gegenstand der vorliegenden Erfindung gehörendes Ausführungsbeispiel eines Behälterverschlusses beim Einsetzen einer Dosierpipette.

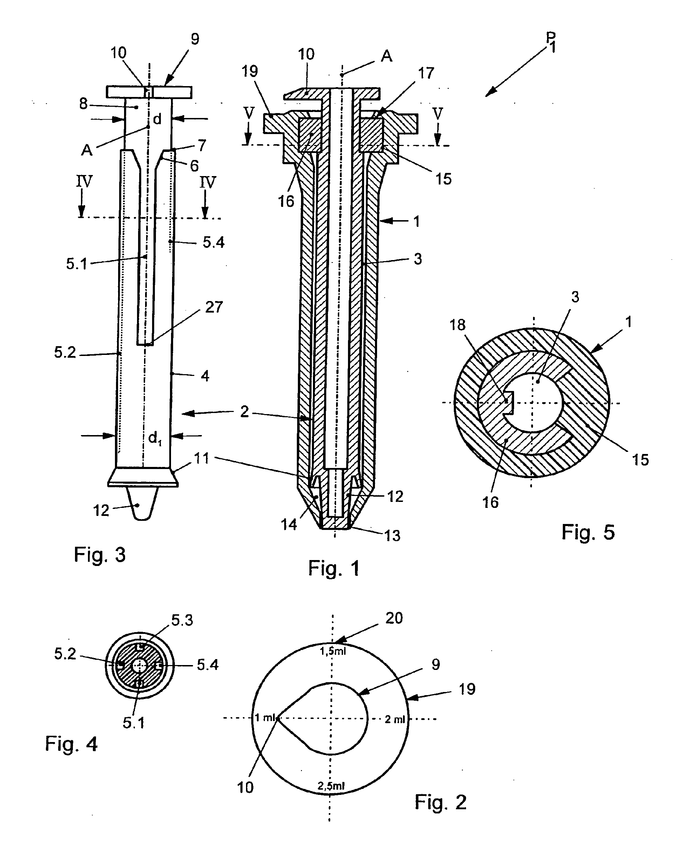

Gemäss Figur 1 weist eine Dosierpipette P₁ ein Dosiergehäuse 1 auf, in welches ein Kolben 2 eingesetzt ist. Der Kolben 2 sitzt in einer Axialausnehmung 3 des Dosiergehäuses 1 und ist in dieser Axialausnehmung 3 drehbar und entlang einer Achse A bewegbar.

Der Kolben 2 weist gemäss Figur 3 und 4 einen Kolbenmantel 4 auf, in den eine Mehrzahl von Rinnen 5.1 bis 5.4 eingeformt sind. Jede dieser Rinnen 5.1 bis 5.4 weist einen Rinneneinlauf 6 auf, der konisch geformt ist.

Nach oben hin bildet der Kolbenmantel 4 eine Stufe 7 aus, an die sich ein Hals 8 anschliesst, wobei ein Durchmesser d des Halses 8 kleiner ist als ein Durchmesser d₁ des Kolbens 2 im Bereich des Kolbenmantels 4. Auf diesen Hals 8 ist eine Scheibe 9 mit einer Nase 10 (siehe Figur 2) aufgesetzt. Diese Scheibe 9 kann als Kopf beliebig ausgebildet und auch dem Hals 8 aufgesetzt oder diesem einstückig angeformt sein.

Nach unten hin schliesst an den Kolbenmantel 4 eine Ringlippe 11 an, die sich nach aussen konisch erweitert. Sie umfängt dabei teilweise einen Endzapfen 12.

Das Dosiergehäuse 1 läuft nach unten hin konisch zu und umfängt dort eine Ansaugöffnung 13. In Schliesslage sitzt der Kolben 2 mit seinem Endzapfen 12 in dieser Ansaugöffnung 13. Die Ringlippe 11 liegt abdichtend der Axialausnehmung 3 an und bildet so zusammen mit dem Dosiergehäuse 1 und dem Endzapfen 12 einen Dosierraum 14 aus, der sich beim Bewegen des Kolbens 2 entlang der Achse A erweitert.

Für die Ausgestaltung des Bereichs der Ansaugöffnung 13 sind mehrere Möglichkeiten denkbar, je nach dem, welche Anwendung die Dosierpipette findet. Die Ausgestaltung kann für eine Vaginalanwendung eine andere sein, als für Rektalanwendung. Ist die Dosierpipette als Salbenapplikator gedacht, so kann eine kugelige Geometrie Anwendung finden, damit keine Verletzungen zugefügt werden. Ferner kann bspw. auch der Endzapfen 12 mit einem Aussengewinde belegt werden, damit er in ein entsprechendes Innengewinde eines Behälters eingeschraubt werden kann. Ein derartiges Aussengewinde kann auch dazu dienen, bspw. einen kugeligen Bereich aufzuschrauben, der, wie oben erwähnt, Verletzungen vermeidet. Denkbar ist auch das Aufsetzen eines Hülsenabschnitts mit einem Innengewinde, das dann den Endzapfen 12 umgibt, wobei das Innengewinde dazu geeignet ist, auf ein Aussengewinde einer Tube aufgeschraubt zu werden. Der Endzapfen 12 greift dann in die Tubenöffnung ein. Hier sind viele Möglichkeiten denkbar und sollen von der vorliegenden Erfindung umfasst sein.

Am gegenüberliegenden Ende ist in einer Gehäuseerweiterung 15 des Dosiergehäuses 1 ein Anschlagring 16 eingelegt und durch einen Bördelrand 17 gehalten. Von diesem Anschlagring 16 ragt, wie in Figur 5 gezeigt, in die lichte Weite der Axialausnehmung 3 des Dosiergehäuses 1 ein Nocken 18 ein, der in Gebrauchslage in eine der Rinnen 5.1 bis 5.4 des Kolbens 2 einfährt.

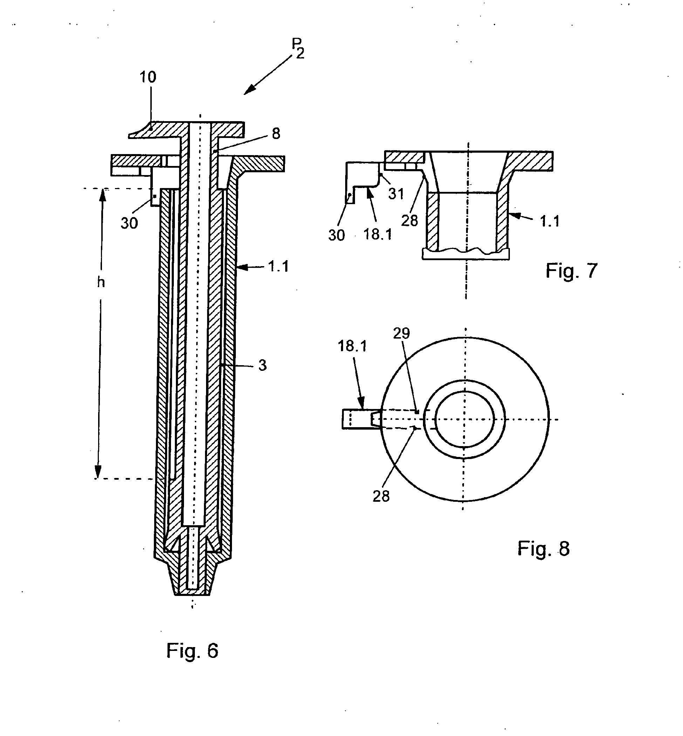

Auf einem Gehäuseflansch 19 befinden sich Markierungen 20, welche eine Angabe über die Dosiermenge enthalten. Diese Dosiermenge hängt von einem Hub h ab, der in Figur 6 angedeutet ist.

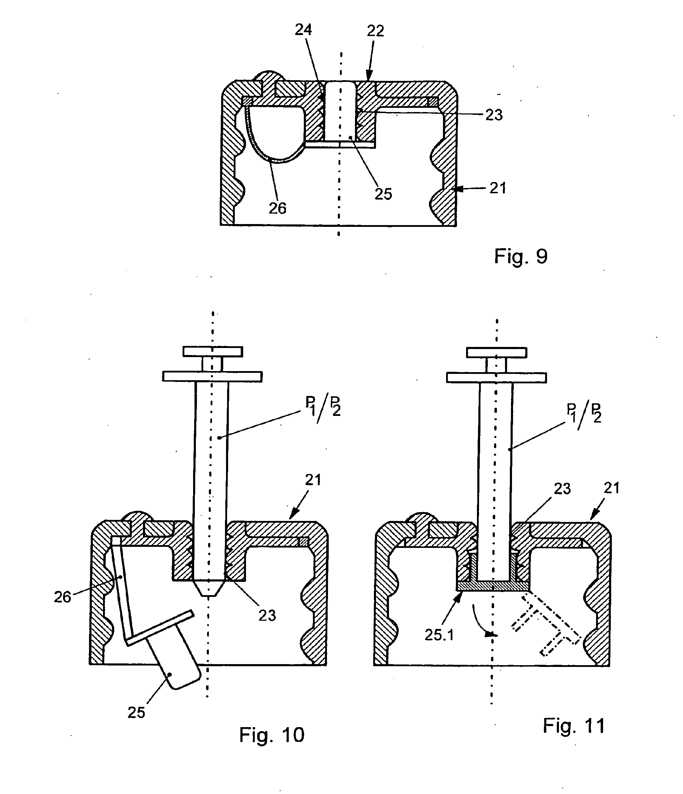

Die Funktionsweise der vorliegenden Erfindung wird insbesondere auch anhand der Figuren 9 bis 11 in kombination mit einem nicht beanspruchten Behälter erörtert:
Ein nicht näher gezeigter Behälter für beispielsweise eine medizinische Flüssigkeit wird durch einen Behälterverschluss 21 verschlossen. Beispielsweise kann dies ein Schraubverschluss sein. In diesem Behälterverschluss 21 befindet sich ein Einsatz 22, der vorzugsweise aus einem weicheren Kunststoff hergestellt ist, wie der Behälterverschluss 21. In diesen Einsatz 22 ist eine Öffnung 23 eingeformt, die vorzugsweise mit Kapillarrillen 24 belegt ist. In der Öffnung 23 sitzt ein Verschlussstopfen 25, der über einen Sicherungsbügel 26 mit dem Einsatz 22 unverlierbar verbunden ist.
Zum Entnehmen einer vorbestimmten Menge an Medium aus dem Behälter wird die Dosierpipette P₁, wie in Figur 10 gezeigt, in die Öffnung 23 eirigestossen, wobei zuvor oder auch danach die Nase 10 des Kolbens 2 auf die gewünschte Markierung 20, welche das Dosiervolumen bestimmt, eingestellt worden ist. Beim Einschieben der Dosierpipette P₁ in die Öffnung 23 wird der Verschlussstopfen 25 ausgestossen und bleibt im Inneren des Behälterverschlusses 21, wie in Figur 10 gezeigt, hängen.

Nunmehr wird der Behälter zusammen mit dem Behälterverschluss und der Dosierpipette über Kopf gestellt und der Kolben 2 in der Axialausnehmung 3 in Richtung der Achse A bewegt, wodurch das im Behälter vorhandene Medium in einem gewünschten Dosiervolumen in den Dosierraum 14 eingesaugt wird. Während dem Bewegen des Kolbens 2 fährt der Nocken 18 der vorbestimmten Rinne 5.1 bis 5.4 entlang, bis er am Grund 27 einer Rinne anschlägt. Hierdurch ist sein Hub h begrenzt, was gleichzeitig bedeutet, dass die gewünschte Menge an Medium sich in dem Dosierraum 14 befindet.

Die Pipette wird jetzt aus der Öffnung 23 gezogen und der Kolben 2 wird zum Ausstossen des Mediums aus dem Dosierraum 14 in entgegengesetzter Richtung entlang der Achse A bewegt, wodurch sich das Volumen des Dosierraums 14 verkleinert. Dabei dichtet die Ringlippe 11 den Dossierraum 14 ab, so dass das gesamte Medium durch die Ansaugöffnung 13 ausgestossen werden kann.

Gegen Ende dieser Bewegung fährt der Endzapfen 12 in die Ansaugöffnung 13 ein und verschliesst diese hermetisch nach aussen, so dass eine bakterielle Verkeimung im System ausgeschlossen wird.

Wird eine andere Dosiermenge gewünscht, wird der Kolben 2 um die Achse A gedreht und die Nase 10 auf die gewünschte Markierung 20 eingestellt. Dies bedeutet, dass dann der Nocken 18 eine andere Rinne 5.1 bis 5.4 abläuft, die eine andere Länge hat und dem gewünschten Dosiervolumen entspricht.

In Figur 11 ist gezeigt, dass ein Verschlussstopfen 25.1 auch verlierbar in der Öffnung 23 angeordnet sein kann. Wenn hier die Dosierpipette P₁ in die Öffnung 23 eingestossen wird, fällt der Verschlussstopfen 25.1, wie gestrichelt angedeutet, in das Behälterinnere und kann dort beispielsweise zum Aufmischen des Mediums beim Schütteln des Behälters verwendet werden.

Eine Dosierpipette P₂, wie in den Figuren 6 bis 8 gezeigt, unterscheidet sich von der Dosierpipette P₁ dadurch, dass der Nocken nicht ein Teil eines Anschlagringes ist, sondern als separates Element ausgebildet ist. Dieser Nocken 18.1 wird seitlich radial in eine Ausnehmung 28 in einem Dosiergehäuse 1.1 eingeschoben, wobei dieses Einschieben durch entsprechende Führungsschienen, die nicht gezeigt sind, unterstützt werden kann. Dabei ist in der Ausnehmung 28 für den Nocken 18.1 eine Rasterung 29 ausgebildet, die in Endlage des Nockens 18.1, bei dem dieser, wie in Figur 6 gezeigt, mit einem Anschlag 30 am Dosiergehäuse 1.1 anschlägt, den Nocken 18.1. hintergreift. In dieser Endlage sitzt der Nocken 18.1 so in der Ausnehmung, dass seine Stirnfläche 31 dem Hals des Kolbens 3 anliegt und so in die Rinnen 5.1 bis 5.4 eingleiten kann.

### Bezugszeichenliste

| | | | | | |
|---|---|---|---|---|---|
| 1 | Dosiergehäuse | 34 | | 67 | |
| 2 | Kolben | 35 | | 68 | |
| 3 | Axialausnehmung | 36 | | 69 | |
| 4 | Kolbenmantel | 37 | | 70 | |
| 5 | Rinnen | 38 | | 71 | |
| 6 | Rinneneinlauf | 39 | | 72 | |
| 7 | Stufe | 40 | | 73 | |
| 8 | Hals | 41 | | 74 | |
| 9 | Scheibe | 42 | | 75 | |
| 10 | Nase | 43 | | 76 | |
| 11 | Ringlippe | 44 | | 77 | |
| 12 | Endzapfen | 45 | | 78 | |
| 13 | Ansaugöffnung | 46 | | 79 | |
| 14 | Dosierraum | 47 | | | |
| 15 | Gehäuseerweiterung | 48 | | | |
| 16 | Anschlagring | 49 | | | |
| 17 | Bördelrand | 50 | | | |
| 18 | Nocken | 51 | | | |
| 19 | Gehäuseflansch | 52 | | | |
| 20 | Markierung | 53 | | | |
| 21 | Behälterverschluss | 54 | | | |
| 22 | Einsatz | 55 | | | |
| 23 | Öffnung | 56 | | | |
| 24 | Kapillarrillen | 57 | | | |
| 25 | Verschlussstopfen | 58 | | | |
| 26 | Sicherungsbügel | 59 | | | |
| 27 | Grund | 60 | | | |
| 28 | Ausnehmung | 61 | | | |
| 29 | Rasterung | 62 | | | |
| 30 | Anschlag | 63 | | | |
| 31 | Stirnfläche | 64 | | | |
| 32 | | 65 | | | |
| 33 | | 66 | | | |

## Patentansprüche

1. Dosierpipette zum dosierten Ausbringen eines Mediums aus einem Behälter mit einem Dosiergehäuse (1) in dem ein Kolben (2) begrenzt axial verschiebbar sitzt, wobei diese Hubbegrenzung durch Drehen des Kolbens (2) veränderbar ist,
**dadurch gekennzeichnet,**
**dass** die Hubbegrenzung durch ein Zusammenwirken eines dem Dosiergehäuse zugeordneten Nockens (18, 18.1) mit unterschiedlich langen Rinnen (5.1 - 5.4) erfolgt, welche axial in einen Kolbenmantel (4) des Kolbens (2) eingeformt sind, wobei der Nocken (18, 18.1) und die Rinnen (5.1 - 5.4) eine Verdrehsicherung ausbilden.

2. Dosierpipette nach Anspruch 1, **dadurch gekennzeichnet, dass** zumindest eine Rinne (5.1 - 5.4) einen Rinneneinlauf (6) aufweist.

3. Dosierpipette nach Anspruch 1 oder 2, **dadurch gekennzeichnet, dass** der Nocken (18) Teil eines Anschlagrings (16) ist, der in das Dosiergehäuse (1) eingesetzt ist.

4. Dosierpipette nach Anspruch 3, **dadurch gekennzeichnet, dass** der Anschlagring (16) in das Dosiergehäuse (1) eingebördelt ist.

5. Dosierpipette nach wenigstens einem der Ansprüche 1 bis 4, **dadurch gekennzeichnet, dass** der Nocken (18.1) In eine radial in dem Dosiergehäuse (1) vorgesehene Ausnehmung (28) eingeschoben ist und in die lichte Weite einer Axialausnehmung (3) des Dosiergehäuses (1) einragte.

6. Dosierpipette nach Anspruch 5, **dadurch gekennzeichnet, dass** der Nocken (18.1) In die Ausnehmung (28) eingeklipst ist.

7. Dosierpipette nach wenigstens einem der Ansprüche 1 bis 6, **dadurch gekennzeichnet, dass** sich der Kolben (2) über eine Ringlippe (11) gegen das Dosiergehäuse (1) abstützt.

8. Dosierpipette nach wenigstens einem der Ansprüche 1 bis 7, **dadurch gekennzeichnet, dass** der Kolben (2) in Schliesslage durch einen Endzapfen (12) eine Ansaugöffnung (13) in dem Dosiergehäuse (1) verschliesst.

9. Dosierpipette nach Anspruch 8, **dadurch gekennzeichnet, dass** der Endzapfen (12) konischen geformt ist.

10. Dosierpipette nach wenigstens einem der Ansprüche 1 bis 9, **dadurch gekennzeichnet, dass** der Kolben (2) zusammen mit dem Dosiergehäuse (1) eine Anzeige für ein Dosiervolumen ausbildet.

11. Dosierpipette nach wenigstens einem der Ansprüche 1 bis 10, **dadurch gekennzeichnet, dass** auf einem oberen Gehäuseflansche (19) des Dosiergehäuses (1) eine Angabe (20) für das Dosiervolumen vermerkt ist, die mit einer Nase (10) an dem Kolben (2) zusammenwirkt.

## Claims

1. Dosing pipette for the metered dispensing of a medium from a container comprising a metering housing (1), in which a plunger (2) is seated axially displaceably in a limited manner, this stroke limitation being variable by rotation of the plunger (2),
**characterised**
**in that** the stroke limitation is effected by an interaction between a cam (18, 18.1) associated with the metering housing and grooves (5.1 - 5.4) of different length which are formed axially in an outer surface (4) of the plunger (2), the cam (18, 18.1) and the grooves (5.1 - 5.4) forming an anti-twist protection.

2. Dosing pipette according to Claim 1, **characterised in that** at least one groove (5.1 - 5.4) has a groove inlet (6).

3. Dosing pipette according to Claim 1 or 2, **characterised in that** the cam (18) is part of a stop ring (16) which is inserted into the metering housing (1).

4. Dosing pipette according to Claim 3, **characterised in that** the stop ring (16) is flanged into the metering housing (1).

5. Dosing pipette according to at least one of Claims 1 to 4, **characterised in that** the cam (18.1) is inserted into a recess (28) provided radially in the metering housing (1) and projects into the clearance of an axial recess (3) of the metering housing (1).

6. Dosing pipette according to Claim 5, **characterised in that** the cam (18.1) is clipped into the recess (28).

7. Dosing pipette according to at least one of Claims 1 to 6, **characterised in that** the plunger (2) is supported against the metering housing (1) by an annular lip (11).

8. Dosing pipette according to at least one of Claims 1 to 7, **characterised in that**, in the closed position, the plunger (2) seals an intake opening (13) in the metering housing (1) by an end journal (12).

9. Dosing pipette according to Claim 8, **characterised in that** the end journal (12) is conical.

10. Dosing pipette according to at least one of Claims 1 to 9, **characterised in that** the plunger (2) together with the metering housing (1) forms an indicator for a metering volume.

11. Dosing pipette according to at least one of Claims 1 to 10, **characterised in that** information (20) for the metering volume is located on an upper housing flange (19) of the metering housing (1), said information interacting with a lug (10) on the plunger (2).

## Revendications

1. Pipette de dosage pour la distribution dosée d'un milieu à partir d'un récipient, avec un boîtier de dosage (1) dans lequel se trouve de manière déplaçable axialement de manière limitée un piston (2), cette limitation de course pouvant être modifiée par rotation du piston (2),
**caractérisée par le fait**
**que** la limitation de course a lieu par une coopération entre une came (18, 18.1) associée au boîtier de dosage et des rainures (5.1 - 5.4) de longueurs différentes qui sont formées axialement dans une enveloppe (4) du piston (2), la came (18, 18,1) et les rainures (5.1 - 5.4) formant une sécurité anti-torsion.

2. Pipette de dosage selon la revendication 1, **caractérisée par le fait qu'**au moins une rainure (5.1 - 5.4) présente une entrée de rainure (6).

3. Pipette de dosage selon la revendication 1 ou 2, **caractérisée par le fait que** la came (18) fait partie d'une bague de butée (16) qui est placée dans le boîtier de dosage (1).

4. Pipette de dosage selon la revendication 3, **caractérisée par le fait que** la bague de butée (16) est sertie dans le boîtier de dosage (1).

5. Pipette de dosage selon au moins l'une des revendications 1 à 4, **caractérisée par le fait que** la came (18,1) est introduite dans un évidement (28) prévu radialement dans le boîtier de dosage (1) et s'engageant dans le diamètre nominal d'un évidement axial (3) du boîtier de dosage (1).

6. Pipette de dosage selon la revendication 5, **caractérisée par le fait que** la came (18.1) est encliquetée dans l'évidement (28).

7. Pipette de dosage selon au moins l'une des revendications 1 à 6, **caractérisée par le fait que** le piston (2) s'appuie par l'intermédiaire d'une lèvre annulaire (11) contre le boîtier de dosage (1).

8. Pipette de dosage selon au moins l'une des revendications 1 à 7, **caractérisée par le fait que** le piston (2) ferme, en position de fermeture, par un tenon d'extrémité (12) une ouverture de succion (13) dans le boîtier de dosage (1).

9. Pipette de dosage selon la revendication 8, **caractérisée par le fait que** le tenon d'extrémité (12) est de forme conique.

10. Pipette de dosage selon au moins l'une des revendications 1 à 9, **caractérisée par le fait que** le piston (2) constitue, ensemble avec le boîtier de dosage (1), une indication d'un volume de dosage.

11. Pipette de dosage selon au moins l'une des revendications 1 à 10, **caractérisée par le fait que** sur une bride de boîtier supérieure (19) du boîtier de dosage (1) est marquée une indication (20) du volume de dosage qui coopère avec un bec (10) sur le piston (2).
